# EUROPEAN PATENT SPECIFICATION

(11) **EP 0 626 841 B1**
(45) Date of publication and mention of the grant of the patent: **20.05.1998**
(21) Application number: 93904380.8
(22) Date of filing: 11.02.1993
(51) Int. Cl.: A61H 39/08, A61H 39/00

(54) **TAPPING ACUPUNCTURE APPARATUS**
KLOPFENDE AKUPUNKTUREVORRICHTUNG
APPAREIL POUR ACUPONCTURE PAR TAPOTEMENTS

(30) Priority: 20.02.1992 KR 9202518
(43) Date of publication of application: 07.12.1994
(73) Proprietor: CHOI, Jang, Youn, Kangsugu, Seoul 157-030 (KR)
(72) Inventor: CHOI, Jang, Youn, Kangsugu, Seoul 157-030 (KR)
(74) Representative: Watkins, David
(86) International application number: PCT/KR93/00011
(87) International publication number: WO 93/16672

(56) References cited:
- EP-A- 0 229 200
- AT-B- 379 079
- US-A- 3 957 053
- Soviet Inventions Illustrated, Section S-X, Week 9151, 12 February 1992, Derwent Publications Ltd., London, S05; & SU,A,1641346 (MOSCOW BAUMAN TECH COLL).
- Soviet Inventions Illustrated, Section S-X, Week 8901, 15 February 1989, Derwent Publications Ltd., London, S05; & SU,A,1405847 (MOSC MED STOMATOL).

## Description

### FIELD OF THE INVENTION

The present invention relates to an acupuncture-tapping apparatus. In particularly, it concerns an acupuncture-tapping apparatus comprising an acupuncture-tapping part including an acupuncture-tapping plate attached various needles, a buffer connector, a driving part, a handle and if necessary, an additional support part, which obtains the effects for improving one's physical condition and preventing the diseases.

### BACKGROUND OF THE INVENTION

There has been no special tapping theory in the world before the present inventor established that theory, and the old instruments for tapping has been those made poorly from a bamboo or plastic, which is too simple to compete with the acupuncture-tapping apparatus of the present invention. As well, the old tapping instruments are quite different from the acupuncture-tapping apparatus of the present invention in respect of the function and the structure.

The present invention is based on the theories of physiotherapy which have kept human health such as acupuncture & moxibustion, Buhang(suction therapy after acupuncturing for suction of virulent blood and abnormal force-energy into a jar), finger-pressure(manual) therapy, X-ray or light projecting therapy, electromagnetic therapy.

Although "the tapping method" and the acupuncture-tapping method asserted by the present inventor may be recognized to be entirely unfamiliar since it has never been theorized yet, a tapping method is well known. Massage is a kind of tapping methods. Generally, people tap or massage a pain region in order to remove pain. The very action is a kind of the tapping methods.

However, it is natural that when someone beats the other on the whole body including his head for the purpose of medical treatment, he may feel fear, discomfort, rejection symptom and the like. However, the present inventor found that the acupuncture-tapping therapy changes the abnormal state of a human body into normal one and moreover has an outstanding effect on improving human habitude-physical constitution through the clinical study subjected to myself, my family, and relatives.

An electric simulation device is known from Soviet Inventions Illustrated, Section S-X, Week 9151, 12 February 1992 relating to the Patent document SU-A-1641346. This device uses an electrostatic charge source formed from a friction pair made of materials with differing dielectric permeabilities. Needles protruding from the surface of one of these materials are given an electrostatic charge by relative frictional movement between the materials and the device is moved over the body of a user with a rolling action.

### SUMMARY OF THE INVENTION

The present invention comprises applying repeatedly the needles of an acupuncture-tapping apparatus and controlling the speed together with heat, light, and electromagnetism on skin, peripheral nerve system and blood vessel to exchange, metastasis and reaction between a macro and a micro force-energy as mentioned above, normalizing the abnormal force-energy, the passage of the fluid materials under the skin and in the exchange, metastasis and reaction of materials and force-energy, resulting in recovering the human body to normal one.

It is an object of the present invention to provide an acupuncture-tapping apparatus which improves the functions of each system, organ and tissue of patients into an optimum state, breaking out the traditional methods for directly treating abnormal symptom, pain and the like in order to recover the state of patients to that of a normal person.

It is another object of the present invention to provide an outstanding effect of acupuncture-tapping therapy.

### BRIEF DESCRIPTION OF DRAWING

Figure 1 is a partially sectional view of the electromotive acupuncture-tapping apparatus(100) of the present invention.

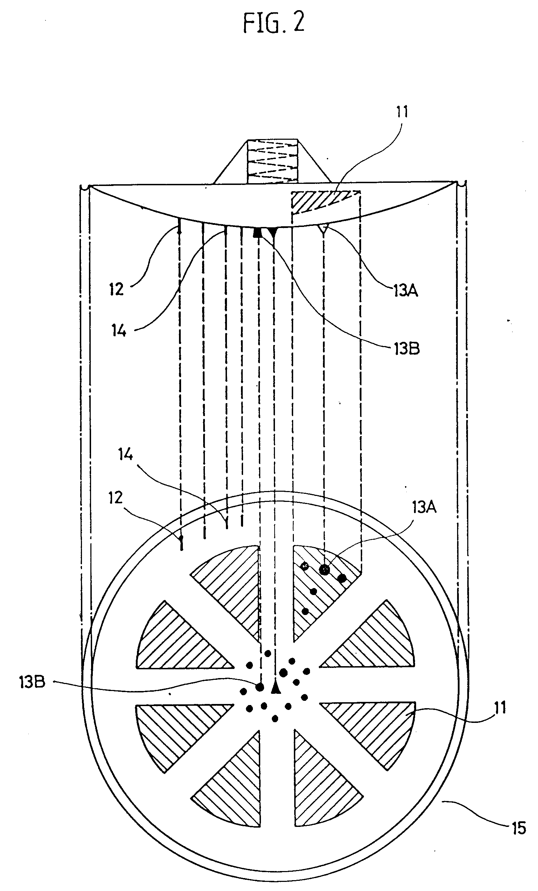

Figure 2 is a side elevational view and a elevational view of the acupuncture- tapping plate(15) of the present invention.

### DETAILED DESCRIPTION OF THE INVENTION

Now, let's assume that a human body is composed of systems, organs and tissues; materials organizing each tissue circulate various path of areas, such as the area where synthesizes and decomposes molecules, the area where atomic physico-chemical reaction, exchange and metastasis occur, the area where the strong interaction, weak interaction, universal gravitation and electromagnetic force exchange with one another, the area where the integrating force is effective, the area of a limited dimensional system where the force-energy of each existence is effective and the area of the system of an unlimited dimension of spirit and mind, the surpassed area(or One Wholeness) without time halt, existence and power, which surpasses cognition and imagination. In this way we can recognize there is always exchange of force-energy between the system of spirit or mind and the system of materials in themselves. Therefore, the definition of diseases in the present invention is the unbalance of the materials of the macro-system(One Wholeness) such as human intestines, muscle, skin, joints, nerves, cells and reacting materials in the human body and the unbalance of energy accompanied pain(ache), a chill, fever, dizziness, lethargy, anxiety, discomfort, insomnia, sleep-walking, illusion, hysteria and the like thereby in the micro-system(One Wholeness). The words such as interruption, superabundance, lack, rupture, interception, connection caused by exchange, reaction and metastasis between materials and the force-energy in human body are consonant with the name of diseasees. Therefore, it is natural that those diseases having such symptoms should be cured in the manner of tracking a path of each unit disease in reverse or regular order.

However, paths of any unit disease from its origin are very complicated and the factors of the micro-system acting on the paths are too many to count, so that the methods selected for prevention and remedy of the unit disease in human body according to the concept of time are countless even among the areas such as medicinal therapy, operational therapy or physical therapy. The reason is that while paths of an unit disease are known in the human body, the paths may be known only as a virtual image outside the human body and the structure and capacity of each system, organ, tissue of the human body are different from individuals. The reason that the paths are recognized as a virtual image is that all the factors of the disease in the human body are delivered to the outside of the human body and they are recognized through the paths of being delivered. Therefore, remedy out of consideration to a size, quality, quantity, speed and the like of the energy of the micro-system is not proper and should be naturally improved.

As we say that petroleum is the very energy, it is natural that we say that a human body is a force, i.e., energy. This expression may be strange enough but it implicates the same meaning. Also, it is so interesting to assume that there are exchange, reaction and metastasis of the microscopic force-energy which man can not see, hear and feel, and moreover such exchange, reaction and metastasis also happen in the limited microscopic force-energy (One Wholeness) that transcends the force-energy mentioned above. There may be an interesting inference when applied those macroscopic materials and microscopic force-energy to individuals.

It is understandable that the total amount of materials and force-energy consumed for the liberation from diseases other than those materials and force-energy consumed only for survival, taste, stamina, promoting health from birth to death will reach into astronomical figures. Instead, if diseases are decreased by the effort to liberate from them, it will be a decisive factor that lengthens the time to consume the limited, usable resources being maintained to mankind.

The history flows toward new and more profitable future and knowledge on the basis of numerous beneficial knowledge accumulated in the past and the present time. The more those who are not healthy and spend a lifetime without emission and absorption of mental energy in a nation are, the weaker the power accumulated in the nation becomes and the more the consumption of usable resources becomes.

The basic theory of the present invention comprises the exchange, reaction and metastasis of the macroscopic force-energy in accordance with tapping acupunctures, and the exchange of the microscopic force energy of heat, light and electromagnetism.

It assumes that men living on one point of the earth receive almost constant eletro-magnetic force throughout his lifetime. For example, it is understandable that since Korean Peninsula is located between 32 ° and 43 ° N. latitude, macrospically it belongs to the scope of the force-energy which is inclined to the direction of the N.pole.

Also, according to one of the ancient theories, the theory of geomancy which is closely related to gravitation and elecromagnetic force, unfortune by water(an oriental theory) can be avoided by selecting Yin(negative) or Yang(positive) and the point on the earth where the quality of earth is optimum and topography is in the optimum balance of life will be found by the same. From such a theory, we can be known how gravitaion and electromagnetic force have been considered and closely applied in real life since olden times and therefore, it gives us even nowadays very precious experience and teaching. However, most people nowadays live without the awareness of the fact that strong and weak interaction, gravitation, and electromagnetic force are related to and have influence on them. The fact that in every moment the force-energy of the external microscopic system exchange energy with the parts of the microscopic human body will give a very significant meaning not only to the scholars who research the force-energy of the microscopic system but also to all people. As a new theory on that, One Wholeness theory that the present inventor believes that the integration of all kinds of concept and thing or force-energy can be derived from the theory of triple-origin circulation is suggested as follows. That is, the theory of triple-origin circulation can be simply described in the drawing;

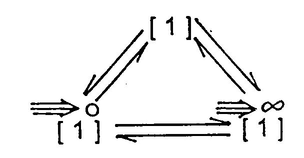

According to the theory, all the knowledge in natural science, human science, and sociology and even the information of civilization can be explained.
[1] The One Wholeness (=Han-Ool Nim=The creative and evolutionary Absolute)
   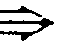 ∘
[1] One Wholeness(It is closed to the mean of once of Wholeness. Infinitesimal-Microscopic System = Han-Ool)
   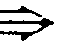 ∞
[1] One Wholeness(It is closed to the mean of once of Wholeness. Universal System = Han-Ool)

When a part of human body is damaged by outer or inner power or a macroscopic disease occurs in a human body, the followings may represent ; changes of the pressure, concentration, distribution, moving speed, temperature and the like of fluid materials such as blood, mallow, hormone, water or aqueous solution comprising aqueous solution, suspension, emulsion or coagulation and the like of an organic or inorganic material in the damaged part, for exampe, in the intestinal area, skin and muscle area, joint area and brain area abnormally occur due to exchange, reaction and metastasis of force-energy. If this state does not recover immediately to a normal state and a long time passes in a state of lacking of its own capacity, the function of the tissues of the damaged part is macrospically decreased and with inertia being accelerated, lastly the unbalance due to the unified system of both a macrosystem and a microsystem can be seen as the symptom of any disease. For instance, it is often seen that when someone's finger is carelessly injured by a hammer having a macroscopic strength, it has a bruise immediately. It is understandable that the damaged part of a human body which is given a considerable force in a moment maintains equilibrium by counteraction and many functions of the human body are self-mobilized to recover the original function. The functions of skin, hypodermic muscle and blood are mostly outstanding and a large amount of blood is mobilzed at the same time. In this process, it is seen that the transferred blood is naturally overloaded, and the blood not recovered from the already mobilized makes the damaged part be swelled and there comes along pain with blood congested while time passes and then the coagulated blood becomes thrombotic and virulent. The recovery of the damaged part without external help takes a very long time. As the fluid materials, fibrous materials and solid materials in the damaged part become abnormal, it is natural that in order to recover rapidly the origninal state or function of the human body, the exchange, metastasis and reaction of force-energy and the fluidity, exchanges and metastasis of fluid materials should be made into normal state.

The present inventor has much experienced in acupuncture medical science and firmly believes that acupunture therapy is never behind medicinal therapy and operational therapy in its effect. WHO(World Health Organization) admitted the 361 regions for acupuncture, 14 meridians about 1000 regions for acupuncture that have been newly established and the thousands of movable regions.

However, the present inventor believes personally that all the parts of a human body can be a region for acupuncture and the force-energy can exchange, metastasis and react on all over the body because the structure and capacity of each system, organ and tissue of individuals differ from each other.

Since the words used in an ancient Oriental medical science are considered to have validity in dealing with the force-energy of micro-system being exchanged in the human body, if the internal exchange, metastasis and reaction make an equality, the meaning of tapping acupuncture therapy will be extended and developed with time passing.

The acupuncture-tapping apparatus (100) according to the present invention comprises an acupuncture-tapping part (10) and a handle 32, characterised in that said apparatus includes an acupuncture-tapping plate (15) having attached needles (11,12,13A,13B,14), a buffer connector (20) attached to the acupuncture-tapping part (10) solely by resilient means (22,23) for buffering the acupuncture-tapping part (10), a driving part (30) attached to the buffer connector (20) for operating the buffer connector (20), said handle (32) including a power switch (35) and current controllers (36,37) for supplying and controlling power to the driving part (30) and a support part (40) connected to the driving part (30) for supporting the driving part (30).

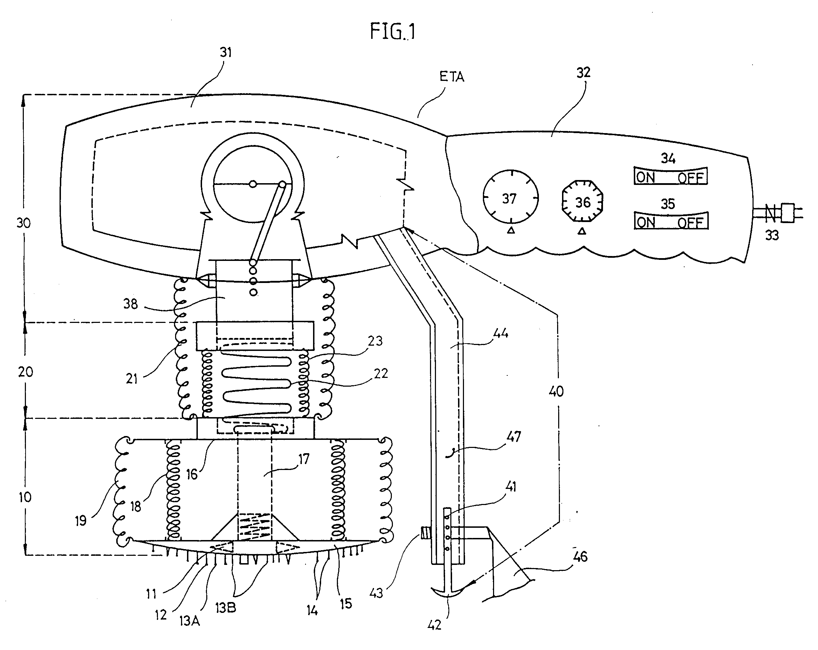

A preferred embodiment of the present invention is represented in Figure 1.

The tapping part (10) of the apparatus of the present invention contains at least four needles per 1cm² of a tapping plate (15) within an area of 200cm² or less, which have a diameter of 0.5 to 5mm and a length of 1 to 10mm.

The needles of the present invention are selected from the group consisting of non-electromagnetic conductive needles (13A), electromagnetic semiconductive needles (13B), electric conductive needles (14), magnetic conductive needles (12), magnetic needles and magnets (11).

The acupuncture-tapping part (10) of the apparatus (100) consists of an upper plate (16) centering around an adjustment screw (17) and a lower plate as an acupuncture-tapping plate (15). The upper plate and the lower plate of the tapping part (10) are connected by buffer springs with each other. The outside of the tapping part(10) is surrounded by a sound- and shock-proof corrugated rubber(19).

The buffer connector(20) of the apparatus(100) according to the present invention consists of a main spring(22) for buffering which links a top of the adjustment screw with a piston(38) of the driving part(30), minor springs (23) between the piston(38) and the tapping part(10) and a sound- and shock-proof corrugated rubber surrounding the outside of the part.

The driving part(30) of the apparatus(100) according to the present invention includes an electromotor(31) as a rotary motion member which, is operated by electricity to make the piston(38) linked with the buffer connector(20) go up and down. The handle(32) of the apparatus(100) includes a power switch(34) of the electromotor, a switch(35) of a light, a current controller(36) of electroconductive needles, a current controller(37) of the electromotor and a main power connecting cord(33).

According to the present invention, a support part(40) is connected to the driving part(30). The support part(40) includes a height-controllable support(44) of which the end has a roller or sliding plate(42) being attached a control screw(41). The support(44) may have a bulb socket(43) for irradiating a light, a screen(46) for covering a light and heat and a fixed hanger (47) of an ice bag.

The method for using the apparatus(100) of the present invention comprises arranging the apparatus(100), i.e., the tapping plate(15) attached various needles on a damaged part or a skin required for tapping in human body, adjusting the roller or sliding plate(44) of the support depending on a desired region and turning on the switches(34, 35) of a current and a light, resulting in a rotary motion of the driving part(30) to move the piston(38) of the driving part(30) upward and downward.

When the piston(38) moves up and down, the sound- and shock-proof corrugated rubber(21) of the buffer connector(20), the main spring and the minor springs(23) move simultaneously so that the motion is transferred to the tapping part(10) through the adjustment screw(17) linked with the buffer connector(20). Accordingly, it causes the tapping plate(15) of the tapping part(10) which is attached various needles to tap the skin.

The time using the acupuncture-tapping apparatus of the present invention may be adjusted depending on the state of the damaged part and the part required for tapping in human body.

Prior to tapping, an oil or vaseline may be applied to the skin.

The feature of the acupuncture-tapping apparatus according to the present invention is to improve the health of the skin and the constitution of the human body on the basis of the acupuncture-tapping therapy using properly the effect of the arrangement, size, number and flexibility of the needles, and the buffer effect, heat, light and electro-magnetic of the tapping plate(15).

When using the acupuncture-tapping apparatus, the tapping starts from hands or feet applying weakly on the part of severe pain until the body accepts the tapping moderately, then increasing intensity until the skin appears to be light pink. The time used in a day is not limited and acupuncture-tapping may apply on the whole body including the damaged part after several days or weeks from the day of the first application of acupuncture.

The tapping therapy of the present invention may use together with warm therapy and cold therapy. The acupuncture-tapping apparatus of the present invention represents stronger stimulation and more immediate effect than the traditional acupuncture apparatus. And for the extensive treatment of the human body, the acupuncture-tapping therapy has more outstanding merits than the existing physical therapy, i.e., acupunture & moxibustion.

In addition, acupuncture-tapping conducted using apparatus according to the present invention excluded rejection symptoms due to the thrill of the needle in the traditional acupuncture therapy and in the bleeding in buhang therapy (extraction of virulent blood into a jar), the shamefulness in finger-pressure (manual) therapy and the side effects in X-ray and electricity-applying therapy.

The tapping therapy carried out with apparatus according to the present invention must be the best news specially for those patients who have diseases of adult people, intractable diseases, diseases requiring a long period to recover and diseases possible to have sequelae, and if even those who think they have no disease apply this tapping acupuncture continuously, they will regain the health of youth and live longer.

The acupuncture-tapping therapy conducted using apparatus according to the present invention may prevent or treat cancers and all the diseases in the systems of skin, respiration, digestion, blood circulation, nerves, muscles and endocrine glands. The acupuncture-tapping therapy has superior effect in improving physical condition and preventing diseases, and the treatment effect is far better than other physical therapies.

The present inventor has clinically confirmed that the effect of acupuncture-tapping therapy carried out with apparatus according to the present invention is better than taking exercise, tonics and medicines for promoting nutrition, in order to prevent diseases.

However, before commencing acupuncture-tapping therapy using apparatus according to the present invention, those who want to improve their physical condition and be cured of their diseases are required to acquire the basic knowledge of the human body and the theory of acupuncture, that is, the basic reasons or principles that the needle is put into the skin so that the painful part and the abnormal system and organ can be cured through the exchange, metastasis and reaction of force-energy.

As explained above, the acupuncture-tapping therapy is a new dimensional therapy which is grafted the acupuncture as one of Oriental medical sciences and the physical therapy as one of modern medical sciences.

The abnormal condition of the human body is recovered by the acupuncture-tapping therapy conducted using apparatus according to the present invention. This helps the exchange of force-energy so as to utilize efficiently a proper quantity of food in the system, organ and tissue of the human body and normalize between materials and the system of materials, materials and the system of force-energy and force-energy and the system of force-energy, not by materials such as tonics, medicines for promoting nutrition. The further effect of the acupuncture-tapping therapy is to protect beneficial microorganisms in the body and change the unidentified microorganisms to have the high adaptability to the body, resulting that weakens and kills harmful microorganisms, enhances immunity against diseases and prevents or delays ageing.

## Claims

1. An acupuncture-tapping apparatus comprising an acupuncture-tapping part (10) and a handle 32, characterised in that said apparatus includes an acupuncture-tapping plate (15) having attached needles (11,12,13A,13B, 14), a buffer connector (20) attached to the acupuncture-tapping part (10) solely by resilient means (22,23) for buffering the acupuncture-tapping part (10), a driving part (30) attached to the buffer connector (20) for operating the buffer connector (20), said handle (32) including a power switch (35) and current controllers (36,37) for supplying and controlling power to the driving part (30) and a support part (40) connected to the driving part (30) for supporting the driving part (30).

2. Apparatus as claimed in Claim 1, wherein the tapping part (10) comprises at least four needles per 1cm² of the tapping plate (15) having an area no greater than 200cm².

3. Apparatus as claimed in Claim 1 or Claim 2, wherein the needles of the tapping part (10) have a diameter of 0.5 to 5 mm and a length of 1 to 10 mm.

4. Apparatus as claimed in any preceding claim, wherein the needles of the tapping plate (15) are selected from the group consisting of non-electromagnetic conductive needles (13A), electromagnetic semiconductive needles (13B), electric conductive needles (14), magnetic conductive needles (12), magnetic needles and magnets (11).

5. Apparatus as claimed in any preceding claim, wherein the support part (40) comprises a support (44) which includes a bulb socket (43) adapted to receive a light bulb.

6. Apparatus as claimed in any preceding claim, wherein the driving part (30) includes a piston (38) which is alternately driven towards and away from said resilient means of said buffer connector (20) for tapping a body.

## Patentansprüche

1. Klopfende Akupunkturvorrichtung, umfassend einen Akupunktur-Klopfteil (10) und einen Handgriff 32, dadurch gekennzeichnet, daß die genannte Vorrichtung folgendes aufweist: eine Akupunktur-Klopfplatte (15) mit daran befestigten Nadeln (11, 12, 13A, 13B, 14), eine Pufferverbindung (20), die an dem Akupunktur-Klopfteil (10) lediglich durch ein elastisches Mittel (22, 23) zum Puffern des Akupunktur-Klopfteils (10) befestigt ist, einen Antriebsteil (30), der an der Pufferverbindung (20) befestigt ist, um die Pufferverbindung (20) zu betreiben, wobei der genannte Handgriff (32) einen Stromschalter (35) und Stromregler (36, 37) beinhaltet, um Strom zum Antriebsteil (30) zu speisen und zu regeln, und einen Abstützteil (40), der mit dem Antriebsteil (30) verbunden ist, um den Antriebsteil (30) zu stützen.

2. Vorrichtung nach Anspruch 1, bei der der Klopfteil (10) wenigstens vier Nadeln pro 1 cm2 der Klopfplatte (15) umfaßt, die eine Fläche von nicht mehr als 200 cm2 hat.

3. Vorrichtung nach Anspruch 1 oder Anspruch 2, bei der die Nadeln des Klopfteils (10) einen Durchmesser von 0,5 bis 5 mm und eine Länge von 1 bis 10 mm haben.

4. Vorrichtung nach einem der vorherigen Ansprüche, bei der die Nadeln der Klopfplatte (15) ausgewählt werden aus der Gruppe bestehend aus nichtelektromagnetischen leitenden Nadeln (13A), elektromagnetischen halbleitenden Nadeln (13B), elektrischen leitenden Nadeln (14), magnetischen leitenden Nadeln (12), magnetischen Nadeln und Magneten (11).

5. Vorrichtung nach einem der vorherigen Ansprüche, bei der der Abstützteil (40) eine Stütze (44) umfaßt, die eine Glühbirnenfassung (43) zur Aufnahme einer Glühbirne aufweist.

6. Vorrichtung nach einem der vorherigen Ansprüche, bei der der Antriebsteil (30) einen Kolben (38) umfaßt, der abwechselnd zu dem elastischen Mittel der genannten Pufferverbindung (20) hin- und von diesem weggetrieben wird, um auf einen Körper zu klopfen.

## Revendications

1. Appareil pour acuponcture par tapotements comprenant une partie d'acuponcture par tapotements (10) et une poignée (32), caractérisé en ce que ledit appareil comprend une plaque d'acuponcture par tapotements (15) sur laquelle sont fixées des aiguilles (11, 12, 13A, 13B, 14), un connecteur tampon (20) fixé à la partie d'acuponcture par tapotements (10) uniquement par des moyens élastiques (22, 23) destiné à amortir la partie d'acuponcture par tapotements (10), une partie d'entraînement (30) fixée au connecteur tampon (20) destinée à actionner le connecteur tampon (20), ladite poignée (32) comprenant un commutateur d'alimentation (35) et des dispositifs de commande de courant (36, 37) destinés à délivrer et commander une puissance à la partie d'entraînement (30), et une partie de support (40) connectée à la partie d'entraînement (30) destinée à supporter la partie d'entraînement (30).

2. Appareil selon la revendication 1, dans lequel la partie d'acuponcture par tapotements (10) comprend au moins quatre aiguilles par 1 cm2 de la plaque d'acuponcture par tapotements (15) ayant une surface ne dépassant pas 200 cm2.

3. Appareil selon la revendication 1 ou la revendication 2, dans lequel les aiguilles de la partie d'acuponcture par tapotements (10) ont un diamètre compris entre 0,5 et 5 mm et une longueur comprise entre 1 et 10 mm.

4. Appareil selon l'une quelconque des revendications précédentes, dans lequel les aiguilles de la plaque d'acuponcture par tapotements (15) sont choisies dans l'ensemble formé par des aiguilles électromagnétiques non conductrices (13A), des aiguilles électromagnétiques semi-conductrices (13B), des aiguilles électriques conductrices (14), des aiguilles magnétiques conductrices (12), aiguilles magnétiques et des aimants (11).

5. Appareil selon l'une quelconque des revendications précédentes, dans lequel la partie de support (40) comprend un support (44) qui comprend une douille d'ampoule (43) adaptée pour recevoir une ampoule.

6. Appareil selon l'une quelconque des revendications précédentes, dans lequel la partie d'entraînement (30) comprend un piston (38) qui est entraîné en va-et-vient vers et loin desdits moyens élastiques dudit connecteur tampon (20) pour tapoter un corps.
